# EUROPÄISCHE PATENTSCHRIFT

(11) **EP 0 503 307 B1**
(45) Veröffentlichungstag und Bekanntmachung des Hinweises auf die Patenterteilung: **17.07.1996**
(21) Anmeldenummer: 92102480.8
(22) Anmeldetag: 14.02.1992
(51) Int. Cl.: A61N 2/04

(54) **Verfahren zur Verwendung einer Magnetdecke, -kissen, -laken, -matraze oder dergleichen**
Method of using a magnetic blanket, cushion, sheet, mattress or like
Méthode d'utilisation d'une couverture, coussin, drap, matelas magnétique ou similaires

(30) Priorität: 15.03.1991 DE 4108437
(43) Veröffentlichungstag der Anmeldung: 16.09.1992
(73) Patentinhaber: Buschky, Rudolf, D-74706 Osterburken (DE); Lüders, Hans-Jürgen, D-29303 Bergen (DE)
(72) Erfinder: Buschky, Rudolf, D-74706 Osterburken (DE)
(74) Vertreter: Clemens, Gerhard, Dr.-Ing.

(56) Entgegenhaltungen:
- EP-A- 0 137 072
- EP-A- 0 332 086
- EP-A- 0 392 626
- DE-A- 3 128 263
- DE-U- 8 518 370
- DE-U- 8 714 410
- FR-A- 2 236 521

## Beschreibung

Die Erfindung betrifft ein Verfahren zur Verwendung einer Magnetdecke, -kissen, -laken, -matratze oder dergleichen mit mehreren in Achsrichtung flachen Induktionsspulen, die achsparallel nebeneinander zwischen Tüchern der Decke oder dergleichen fixiert sind, und mit einem Erregerstromgenerator für diese Spulen.

Wenn hier und im folgenden der Einfachheit halber von einer Magnetdecke die Rede ist, dann ist damit auch ein Magnetkissen, ein Magnetlaken, eine Magnetmatratze oder dergleichen gemeint, ohne daß dies besonders hervorgehoben ist.

Bei einer in der deutschen Patentanmeldung P 4004682 beschriebenen Magnetdecke dieser Art werden Magnetfelder erzeugt, die, bezogen auf ihre Polung, einen Benutzer der Decke in unterschiedlichen Richtungen und auch in wechselnden Richtungen durchsetzen.

Es hat sich gezeigt, daß sensible Benutzer Störungen empfinden, wenn die Polung des durchsetzenden Magnetfeldes gewechselt wird. Ein solcher Polwechsel kann entstehen durch entsprechend wechselnde Erregung der Spulen aber auch dadurch, daß ein Magnetfeld induziert wird, das dem Erdmagnetfeld entgegengerichtet ist. Wird ein solches Magnetfeld ein- und abgeschaltet, dann entsteht dennoch in dem resultierenden Magnetfeld ein Feldwechsel, der auf den Benutzer einwirkt.

Die FR-A-2 236 521 offenbart eine Auflage für einen Patienten, die mittels einem Kompaß in bezug auf das Erdmagnetfeld ausgerichtet werden soll.

Aufgabe der Erfindung ist es, solche Feldwechsel zu vermeiden.

Die Erfindung löst diese Aufgabe dadurch, daß die Erregung aller Spulen mit Bezug auf das erregte Magnetfeld einerseits und mit Bezug auf die gleichgerichtete Komponente des Erdmagnetfeldes gleichpolig erfolgt.

Wenn der Benutzer die Decke horizontal ausbreitet und sich darauf legt, dann ist die durch die Magnetdecke induzierte, den Benutzer durchsetzende Magnetfeldrichtung vertikal. Das Erdmagnetfeld erstreckt sich, abhängig von der geografischen Breite, mit zum Pol zunehmender vertikaler Komponente. Mit der Polung dieser Komponente muß das zu erregende Magnetfeld übereinstimmen, damit nicht durch Ein- und Ausschalten ein Polwechsel stattfindet. Die Polungsbedingungen hierfür sind auf der Nordhalbkugel umgekehrt zu denen auf der Südhalbkugel.

Um sicherzustellen, daß keine Verwechselungen auftreten, empfiehlt es sich, daß eine Markierung für die Polarität des erregten beziehungsweise zu erregenden Magnetfeldes von außen lesbar an der Decke fixiert ist und/oder daß ein Meßgerät mit Richtungsanzeiger für das herrschende Magnetfeld von außen ablesbar an der Decke fixiert ist.

Vorzugsweise sind permanentmagnetische Elemente in der Magnetdecke vorgesehen, die hinsichtlich ihrer magnetischen Polarität gleichsinning zum Erdmagnetfeld ausgerichtet und in der Magnetdecke fixiert sind. Solche permanentmagnetischen Elemente weisen vorzugsweise eine Magnetfeldstärke auf, die etwa das 1- bis 50fache des örtlichen Erdmagnetfeldes beträgt. Auch dadurch kann ein unerwünschter Polwechsel beim Ein- und Ausschalten der Erregung der Induktionsspulen vermieden werden.

Eine Magnetmatratze kann mit einem Federkern ausgestattet sein. Handelt es sich um Stahlfedern, dann haben diese fertigungsbedingt in vielen Fällen einen Permanentmagnetismus. Damit nun durch den von diesen Stahlfedern hervorgerufenen magnetischen Feldern nicht ein unerwünschter Polbeim Ein- und Ausschalten der Induktion stattfindet, ist eine Weiterbildung der Erfindung, die sich auf eine Magnetmatratze bezieht, dadurch gekennzeichnet, daß ein permanentmagnetisches Element eine schraubenförmige Druckfeder aus Stahl ist, die koaxial oder achsparallel zu einer Induktionsspule angeordnet und fixiert ist.

Zur Vermeidung von Fehlschaltungen empfiehlt es sich auch, eine Umpolung der Anschlüsse des Erregerstromgenerators an die Spulen durch nur eindeutig kuppelbar ausgestaltete Stromkupplungen zu verhindern.

In der eingangs erwähnten Patentanmeldung ist beschrieben, die Spulen gruppenweise zusammenzufassen und gruppenweise zeitlich versetzt zueinander zu erregen. Damit erzielt man einen wünschenswerten massageähnlichen Effekt.

Es hat sich gezeigt, daß es günstig ist, diesem Massageeffekt eine zum Herz führende Richtung zu geben, und das wird sehr einfach dadurch erzielbar, daß die Induktionsspulen in Spulengruppen zusammengefaßt sind, wobei jede Spulengruppe über eine gesonderte Anschlußleitung an den Erregerstromgenerator angeschlossen ist, daß der Erregerstromgenerator mehrere Erregerstromausgänge aufweist, die schaltungsmäßig unterschiedlichen Spulengruppen zugeordnet sind, daß die Erregung der einzelnen Spulengruppen mit zeitlichem Versatz erfolgt, derart, daß die in einer Erregerperiode zuerst zu erregende Spulengruppe am Rand der durch die Magnetdecke definierten Funktionsfläche gelegene Spulen aufweist, daß die zuletzt in dieser Erregerperiode zu erregende Spulengruppe im mittleren Bereich der Funktionsfläche gelegene Spulen aufweist und daß eventuelle weitere Spulengruppen dazwischen gelegene Spulen umfassen, deren Erregung zeitlich zum mittleren Bereich fortschreitend erfolgt.

In diesem Fall ist es sehr einfach, den mittleren Bereich der Funktionsfläche der Lage des Herzens eines Benutzers zuzuordnen, indem sich der Benutzer entsprechend positioniert auf die Decke legt. Dazu empfiehlt es sich, die Umrisse einer menschlichen Gestalt in der optimalen Lage, in der sie auf der Decke liegen sollte, auf der Oberseite der Decke anzuzeigen.

Die Erregung kann dabei in Längsrichtung der Funktionsfläche und/oder in Querrichtung der Funktionsfläche zum mittleren Bereich fortschreitend erfolgen. Erleichtert wird die Zuordnung, wenn Markierungen und/oder Anzeigeelemente zur Kennzeichnung der Lage des mittleren Bereichs und/oder der einzelnen Spulengruppen und ihrer Erregung von außen ablesbar an der Decke fixiert sind. Bei den Anzeigeelementen handelt es sich vorzugsweise um Signallampen, die immer dann aufleuchten, wenn die jeweils zugeordnete Induktionsspule magnetisch erregt ist.

Die Erfindung wird nun anhand der beigefügten Zeichnung näher erläutert.

In der Zeichnung zeigt:
- Figur 1: eine Magnetdecke mit einem angeschlossenen Erregerstromgenerator, wobei von der Magnetdecke das dem Beschauer zugekehrte Tuch zum Teil abgebrochen dargestellt ist,
- Figur 2: eine Kupplung für die Stromzuleitung vom Erregerstromgenerator zur Magnetdecke aus Figur 1,
- Figur 3: ein Erregerdiagramm zu Figur 1,
- Figur 4: die Decke aus Figur 1 in Draufsicht mit Markierungen und einem Meßgerät,
- Figur 5: ein Diagramm für eine gegenüber Figur 1 abgeänderte Schaltung der Spulen,
- Figur 6 und 7: je ein Diagramm für eine andere Decke mit anders geschalteten Spulen und
- Figur 8: ausschnittsweise eine Magnetmatratze mit Federkern.

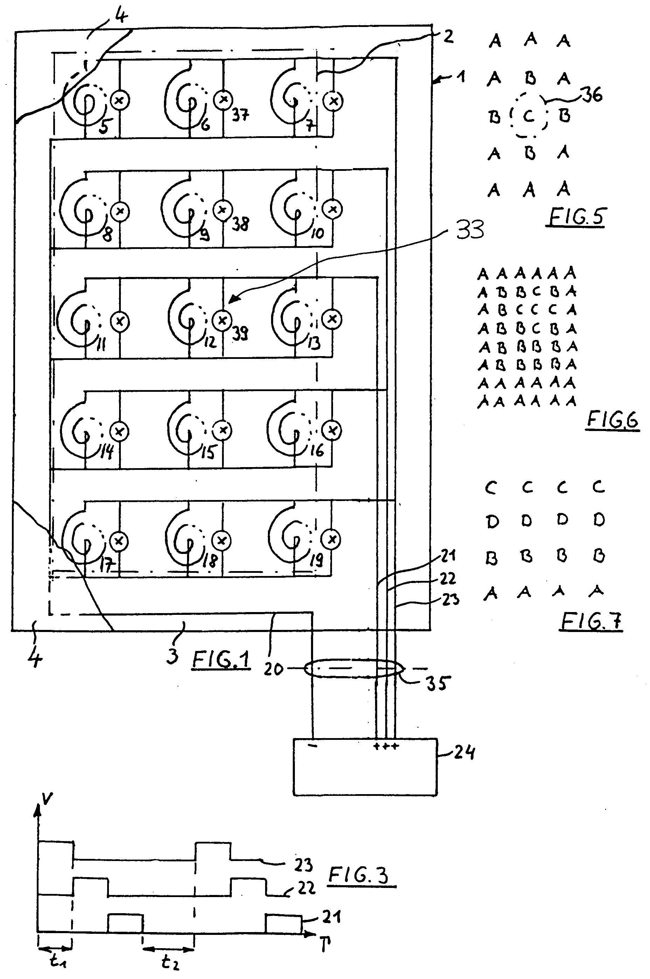

Die in Figur 1 dargestellte Magnetdecke 1 hat eine rechteckige Grundform, die gleichzeitig die Funktionsfläche 2 definiert. Die Magnetdecke besteht aus einem unteren, dem Beschauer von Figur 1 abgekehrten Tuch 3 und einem nur teilweise dargestellten, dem Beschauer von Figur 1 zugekehrten Tuch 4 sowie insgesamt fünfzehn flachen Induktionsspulen 5 bis 19, die achsparallel nebeneinander zwischen den Tüchern fixiert sind.

Die Induktionsspulen 5 bis 19 sind alle mit gleichem Drehsinn angeordnet und gleichpolig über die Minus-Leitung 20 und eine der Plus-Leitungen 21, 22, 23 an einen Erregerstromgenerator 24 angeschlossen. Die Induktionsspulen 5, 6, 7, 17, 18, 19 bilden eine erste Spulengruppe und sind über die Plus-Leitung 21 angeschlossen. Die Induktionsspulen 8, 9, 10, 14, 15, 16 bilden eine zweite Spulengruppe und sind über die Plus-Leitung 22 angeschlossen. Die Induktionsspulen 11, 12, 13 bilden die dritte Spulengruppe und sind über die Plus-Leitung 23 angeschlossen.

Der Erregerstromgenerator 24 erzeugt Gleichstromimpulse wie aus dem Diagramm Fig. 3 ersichtlich, in dem auf der vertikalen Achse die Spannung V und auf der horizontalen Achse die Zeit T aufgetragen ist. Alle Gleichstrom-Impulse haben die gleiche zeitliche Länge t1 von 3 bis 20 Sekunden, vorzugsweise 12 Sekunden.

Eine Erregerperiode beginnt mit einem Impuls t1 auf der Plus-Leitung 21. Wenn dieser Impuls beendet ist, beginnt ein Impuls auf der Plus-Leitung 22. Wenn dieser beendet ist, beginnt ein Impuls auf der Plus-Leitung 23. Wenn dieser beendet ist, beginnt nach einer Pause t2 erneut ein Impuls auf der Plus-Leitung 21 und so fort. Die Pausenzeit t2 ist etwas länger als die Impulsdauer t1. Vorzugsweise beträgt t1 = 5 sec. (Sekunden) und t2 = 7 sec.

Bedingt durch die Polung werden in den erregten Induktionsspulen Magnetfelder erzeugt, deren Feldrichtung Nord aus der Zeichenebene heraus auf den Beschauer von Figur 1 zu gerichtet ist. Dem entspricht die Markierung 30, die auf der Oberseite der Magnetdecke fixiert ist. An der Oberseite der Magnetdecke ist von außen sichtbar ein Kompaß 31 befestigt.

Zur Benutzung wird die Magnetdecke um eine vertikale Achse gedreht, bis eine Lage erreicht ist, in der die Kompaßnadel bei eingeschaltetem und bei ausgeschaltetem Erregerstromgenerator 24 in die gleiche Richtung zeigt. Dann ist sichergestellt, daß in den Impulslücken der Gleichstromimpulse des Erregerstroms keine Polumkehr auf den Benutzer einwirkt. Anstelle des an der Magnetdecke befestigten Kompaß 31 kann man auch einen losen, gesonderten Kompaß verwenden, den man zur Einrichtung der Magnetdecke auf diese oder daneben hält.

Das Magnetfeld entsteht zuerst am oberen und unteren Rand der Decke, oben bezogen auf die Darstellung der Figur 1. Dann entsteht es in einem Zwischenbereich und dann schließlich in einem mittleren Bereich 33, an der sich etwa das Herz einer auf der Decke liegenden Benutzerperson befinden sollte. Um diese Lage der Benutzerperson anzuzeigen, ist die Markierung 43 auf der Oberseite der Decke sichtbar aufgetragen. Außerdem ist die Anordnung der Induktionsspulen durch Buchstabenmarkierungen 40 bis 42 .... auf der Oberfläche der Magnetdecke gekennzeichnet. Den einzelnen Buchstaben sind Signallampen 37 bis 39 zugeordnet, die sich auf der Oberseite der Decke genau über dem Zentrum einer jeweils zugeordneten Induktionsspule befinden. Diese Signallampen sind, wie aus Figur 1 ersichtlich, elektrisch mit den zugehörigen Induktionsspulen parallel geschaltet und leuchten immer dann auf, wenn die zugehörige Induktionsspule erregt ist.

Für alle Induktionsspulen sind Buchstaben von A bis C angebracht, und zwar für die Induktionsspulen der Plus-Leitung 21 der Buchstabe A, für die Induktionsspulen der Plus-Leitung 22 der Buchstabe B und für die Induktionsspulen der Plus-Leitung 23 der Buchstabe C.

Mit 35 ist eine lösbare Kupplung für die Leitung 20 bis 23 vorgesehen, die, um sicherzustellen, daß die Polung nicht unbeabsichtigt geändert wird, so ausgestaltet ist, daß sie nur eindeutig, das heißt nur in einer, nämlich der richtigen, Kuppelstellung einkuppelbar ist. Wie aus Figur 2 ersichtlich sind insgesamt vier Stecker und vier Buchsen vorgesehen, die unsymmetrisch angeordnet sind, so daß sie nur in der einen, richtigen Stellung kuppelbar sind.

In Abänderung des Ausführungsbeispiels nach Figur 1 bis 4 können die dort vorgesehenen Induktionsspulen auch in anderer Weise in drei Spulen-Gruppen zusammengefaßt werden, wie dies durch das Buchstabendiagramm aus Figur 5 angedeutet ist. Das Magnetfeld läuft dann in einer Periode nicht nur in Längsrichtung der Decke, sondern auch in Querrichtung der Decke auf den mittleren Bereich 36 zu, der durch eine strichpunktierte Linie angedeutet ist.

Bei größer Anzahl von Magnetspulen kann man diese auch zu mehr als drei Spulen-Gruppen zusammenfassen, wie dies beispielsweise in Figur 6 durch die Buchstabenfolge A - B - C angedeutet ist.

Bei dem in Figur 7 dargestellten Ausführungsbeispiel sind die insgesamt zwölf vorgesehenen Induktionsspulen in vier Spulengruppen zusammengefaßt entsprechend den eingezeichneten Buchstaben, wobei die Erregung in der Buchstabenfolge A - B - C - D erfolgt.

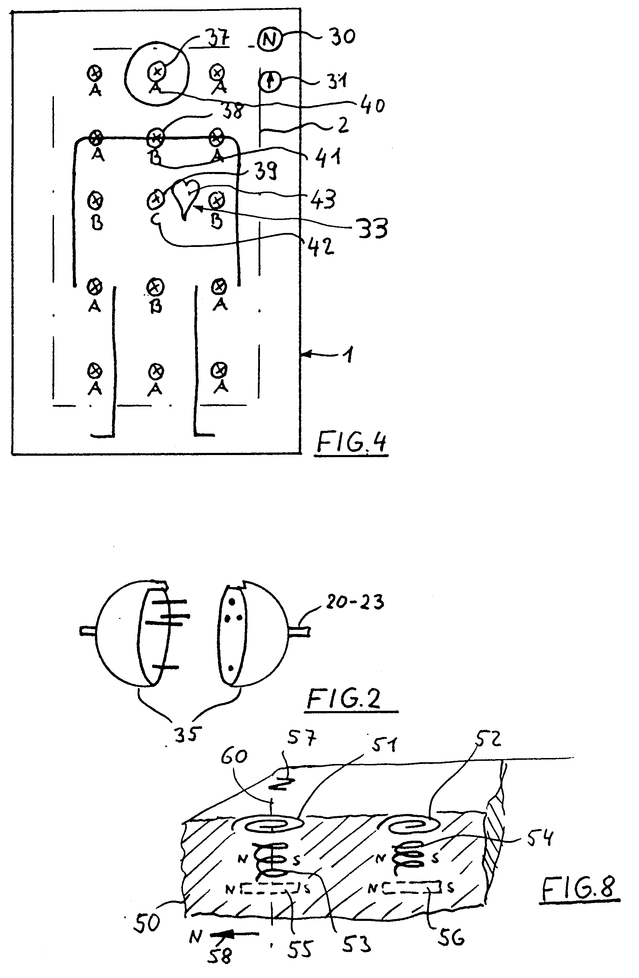

Figur 8 zeigt ausschnittsweise im Teilschnitt eine Magnetmatratze 50 mit Federkern. Der Federkern besteht aus mehreren über die ganze Matratzenausdehnung verteilten, schraubenförmigen Spiralfedern 53, 54, die koaxial zu Induktionsspulen 51, 52 angeordnet und in der Matratze fixiert sind. Die Induktionsspulen sind so angeordnet und geschaltet wie im Text zu Figur 1 beschrieben. Es sind also insgesamt wie nach Figur 1 fünfzehn Induktionsspulen vorgesehen und dementsprechend auch fünfzehn Spiralfedern, von denen jedoch nur die Spiralfedern 53, 54 in der Zeichnung sichtbar sind.

Die Spiralfedern 53, 54, die aus Stahl bestehen und schraubenförmig gewunden sind, haben einen Permanentmagnetismus. Sie werden beim Einbau durch Drehen um ihre Achse 60 so ausgerichtet, daß dieser Permanentmagnetismus in die Nordrichtung gemäß Pfeil 58 zeigt, wenn die Matratze 50 entsprechend dem Symbol 57 in Nordrichtung ausgerichtet ist. Entsprechendes gilt auch für die Spiralfeder 54 und die übrigen nicht dargestellten Druckfedern.

In Abänderung des dargestellten Ausführungsbeispiels können weitere Spiralfedern zwischen den Induktionsspulen angeordnet sein, die dann gleichmäßig über die Fläche der Magnetmatratze verteilt sind und achsparallel zur Achse 60 angeordnet sind. Die magnetische Ausrichtung erfolgt entsprechend wie zur Spiralfeder 53 erläutert.

In einer weiteren Abänderung des dargestellten Ausführungsbeispiels können auch einfache permanentmagnetische Elemente, zum Beispiel permanentmagnetische Stäbe wie die Magnetstäbe 55 und 56 in der Magnetmatratze angeordnet und fixiert sein. Sie sind dann bezüglich ihrer magnetischen Polung ebenfalls wie die Spiralfedern nach dem Erdmagnetismus gemäß Pfeil 58 ausgerichtet. Die magnetische Feldstärke der Spiralfedern 53, 54 beziehungsweise der Magnetelemente 55, 56 beträgt etwa das 10fache der örtlichen Feldstärke des erdmagnetischen Feldes.

Die Orientierung der Matratze im Raum erfolgt mit Hilfe eines Kompaß wie im Text zu Figur 1 bis 7 beschrieben.

## Patentansprüche

1. Verfahren zur Verwendung einer/eines Magnetdecke, -kissen, -laken, -matratze oder dergleichen
mit mehreren in Achsrichtung flachen Induktionsspulen, die achsparallel nebeneinander zwischen Tüchern der Decke oder dergleichen fixiert sind, und
mit einem Erregerstromgenerator für diese Spulen, dadurch gekennzeichnet, daß die Magnetdecke derart ausgerichtet wird,
daß die Erregung aller Spulen ( 5 - 19 ) mit Bezug auf das erregte Magnetfeld einerseits und mit Bezug auf die gleichgerichtete Komponente des Erdmagnetfeldes gleichpolig erfolgt.

2. Verfahren nach Anspruch 1, dadurch gekennzeichnet,
daß eine Markierung ( 30 ) für die Polarität des erregten beziehungsweise zu erregenden Magnetfeldes von außen lesbar an der Decke ( 1 ) fixiert ist.

3. Verfahren nach Anspruch 1, dadurch gekennzeichnet,
daß ein Meßgerät mit Richtungsanzeiger ( 31 ) für das herrschende Magnetfeld von außen ablesbar an der Decke ( 1 ) fixiert ist.

4. Verfahren nach einem der vorhergehenden Ansprüche, dadurch gekennzeichnet,
daß permanentmagnetische Elemente ( 53 - 56 ) in der Magnetdecke ( 50 ) vorgesehen sind, die hinsichtlich ihrer magnetischen Polarität gleichsinning zum Erdmagnetfeld ausgerichtet und in der Magnetdecke fixiert sind.

5. Verfahren nach Anspruch 4, dadurch gekennzeichnet,
daß ein permanentmagnetisches Element ( 53, 54 ) eine schraubenförmige Druckfeder aus Stahl ist, die koaxial oder achsparallel zu einer Induktionsspule ( 51, 52 ) angeordnet und fixiert ist.

6. Verfahren nach einem der vorhergehenden Ansprüche, dadurch gekennzeichnet,
daß die Induktionsspulen ( 5 - 19 ) in Spulengruppen ( A, B, C ) zusammengefaßt sind, wobei jede Spulengruppe über eine gesonderte Anschlußleitung ( 21, 22, 23) an den Erregerstromgenerator ( 24 ) angeschlossen ist,
daß der Erregerstromgenerator mehrere Erregerstromausgänge aufweist, die schaltungsmäßig unterschiedlichen Spulengruppen zugeordnet sind, und
daß die Erregung der einzelnen Spulengruppen mit zeitlichem Versatz erfolgt, derart, daß die in einer Erregerperiode zuerst zu erregende Spulengruppe am Rand ( A ) der durch die Magnetdecke definierten Funktionsfläche ( 2 ) gelegene Spulen aufweist,
daß die zuletzt in dieser Erregerperiode zu erregende Spulengruppe ( C ) im mittleren Bereich ( 33 ) der Funktionsfläche gelegene Spulen aufweist und
daß eventuelle weitere Spulengruppen ( B ) dazwischen gelegene Spulen umfassen, deren Erregung zeitlich zum mittleren Bereich fortschreitend erfolgt.

7. Verfahren nach Anspruch 5, dadurch gekennzeichnet,
daß der mittlere Bereich ( 33 ) der Funktionsfläche ( 2 ) der Lage des Herzens eines Benutzers zugeordnet ist.

8. Verfahren nach Anspruch 5 oder 6, dadurch gekennzeichnet,
daß die Erregung in Längsrichtung der Funktionsfläche zum mittleren Bereich fortschreitend erfolgt.

9. Verfahren nach Anspruch 5 oder 6, dadurch gekennzeichnet,
daß die Erregung in Querrichtung der Funktionsfläche zum mittleren Bereich fortschreitend erfolgt.

10. Verfahren nach einem der vorhergehenden Ansprüche, dadurch gekennzeichnet,
daß Markierungen ( 40 - 43 ) und/oder Anzeigeelemente ( 37 - 39 ) zur Kennzeichnung der Lage des mittleren Bereichs ( 33 ) und/oder der einzelnen Spulengruppen und ihrer Erregung von außen ablesbar an der Decke ( 1 ) fixiert sind.

## Claims

1. Method of using a magnetic blanket, cushion, sheet, mattress or the like, having a plurality of induction coils which are flat in the axial direction and are fixed axially parallel alongside one another between cloths of the blanket or the like, and having an excitation current generator for these coils, characterized in that the magnetic blanket is aligned in such a way that the excitation of all the coils (5 - 19) is carried out with the same polarity in relation to the excited magnetic field, on the one hand, and in relation to the similarly directed component of the earth's magnetic field.

2. Method according to Claim 1, characterized in that a marking (30) for the polarity of the excited magnetic field or of the magnetic field to be excited is fixed to the blanket (1) so as to be legible from outside.

3. Method according to Claim 1, characterized in that a measuring device with a direction indicator (31) for the prevailing magnetic field is fixed to the blanket (1) so as be legible from outside.

4. Method according to one of the preceding claims, characterized in that permanent magnetic elements (53 - 56) are provided in the magnetic blanket (50), the said elements being aligned, with respect to their magnetic polarity, in the same direction as the earth's magnetic field and being fixed in the magnetic blanket.

5. Method according to Claim 4, characterized in that a permanent magnetic element (53, 54) is a helical compression spring made of steel, which is arranged and fixed coaxially or axially parallel to an induction coil (51, 52).

6. Method according to one of the preceding claims, characterized in that the induction coils (5 - 19) are combined into coil groups (A, B, C), each coil group being connected to the excitation current generator (24) via a separate connecting line (21, 22, 23), in that the excitation current generator has a plurality of excitation current outputs which are assigned in terms of circuitry to different coil groups, and in that the excitation of the individual coil groups is carried out with a time offset in such a way that the coil group to be excited first in one excitation period has coils placed at the edge (A) of the functional area (2) defined by the magnetic blanket, in that the coil group (C) to be excited last in this excitation period has coils placed in the central region (33) of the functional area, and in that any further coil groups (B) comprise coils which are placed therebetween and whose excitation is carried out progressively in time towards the central region.

7. Method according to Claim 5, characterized in that the central region (33) of the functional area (2) is assigned to the position of the heart of a user.

8. Method according to Claim 5 or 6, characterized in that the excitation is carried out progressively towards the central region in the longitudinal direction of the functional area.

9. Method according to Claim 5 or 6, characterized in that the excitation is carried out progressively towards the central region in the transverse direction of the functional area.

10. Method according to one of the preceding claims, characterized in that markings (40 - 43) and/or indicating elements (37 - 39) for identifying the position of the central region (33) and/or of the individual coil groups and their excitation are fixed to the blanket (1) so as to be legible from outside.

## Revendications

1. Procédé d'utilisation d'une couverture, coussin, drap, matelas magnétique ou similaires, avec plusieurs bobines d'induction plates en direction axiale, qui sont fixées en parallélisme axial les unes à côté des autres entre des tissus de la couverture ou similaires, et avec un générateur de courant d'excitation pour ces bobines, **caractérisé** en ce que la couverture magnétique est orientée de telle sorte que l'excitation de toutes les bobines (5 à 19) s'effectue avec la même polarité par rapport au champ magnétique excité d'une part et par rapport à la composante redressée du champ magnétique terrestre.

2. Procédé selon la revendication 1, **caractérisé** en ce qu'un repère (30), lisible de l'extérieur, est fixé sur la couverture (1) pour la polarité du champ magnétique excité ou à exciter.

3. Procédé selon la revendication 1, **caractérisé** en ce qu'un appareil de mesure avec indicateur de direction (31) lisible de l'extérieur pour le champ magnétique régnant est fixé sur la couverture (1).

4. Procédé selon une des revendications précédentes, **caractérisé** en ce que des éléments à magnétisation permanente (53 à 56) sont prévus dans la couverture magnétique (50), éléments qui sont fixés dans la couverture magnétique en étant orientés, en ce qui concerne leur polarité magnétique, dans le même sens par rapport au champ magnétique terrestre.

5. Procédé selon la revendication 4, **caractérisé** en ce qu'un élément à magnétisation permanente (53, 54) est un ressort de pression en hélice en acier, qui est disposé et fixé coaxialement ou parallèlement à l'axe d'une bobine d'induction (51, 52).

6. Procédé selon une des revendications précédentes, **caractérisé** en ce que les bobines d'induction (5 à 19) sont réunies en groupes de bobines (A, B, C), chaque groupe de bobines étant raccordé au générateur de courant d'excitation (24) par une ligne de raccordement séparée (21, 22, 23), en ce que le générateur de courant d'excitation présente plusieurs sorties de courant d'excitation qui sont associées en matière de connexion à des groupes de bobines différents, et en ce que l'excitation des différents groupes de bobines s'effectue avec un décalage dans le temps de telle sorte que le groupe de bobines à exciter en premier dans une période d'excitation présente des bobines situées sur le bord (A) de la face fonctionnelle (2) définie par la couverture magnétique, que le groupe de bobines (C) à exciter en dernier dans cette période d'excitation présente des bobines situées dans la région médiane (33) de la face fonctionnelle, et que d'éventuels autres groupes de bobines (B) présentent des bobines situées dans les régions intermédiaires, dont l'excitation s'effectue avec une progression dans le temps vers la région médiane.

7. Procédé selon la revendication 5, **caractérisé** en ce que la région médiane (33) de la face fonctionnelle (2) est associée à la position du coeur d'un utilisateur.

8. Procédé selon la revendication 5 ou 6, **caractérisé** en ce que l'excitation s'effectue avec une progression vers la région médiane dans la direction longitudinale de la face fonctionnelle.

9. Procédé selon la revendication 5 ou 6, **caractérisé** en ce que l'excitation s'effectue avec une progression vers la région médiane dans la direction transversale de la face fonctionnelle.

10. Procédé selon une des revendications précédentes, **caractérisé** en ce que des repères (40 à 43) et/ou des éléments indicateurs (37 à 39), lisibles de l'extérieur, sont fixés sur la couverture (1) pour identifier la position de la région médiane (33) et/ou des différents groupes de bobines et de leur excitation.
